# FASCICULE DE BREVET EUROPEEN

(11) **EP 1 143 911 B1**
(45) Date de publication et mention de la délivrance du brevet: **08.01.2003**
(21) Numéro de dépôt: 99934814.7
(22) Date de dépôt: 29.07.1999
(51) Int. Cl.: A61K 6/00

(54) **MATERIAU D'OBTURATION UTILISE APRES UNE PULPOTOMIE**
FÜLLMATERIAL ZUR VERWENDUNG NACH EXTRAKTION VON LEBENDER PULPA DENTIS
PASTY COMPOSITION FOR USE AS SEALING MATERIAL IN PULPOTOMY

(30) Priorité: 29.07.1998 FR 9809712
(43) Date de publication de la demande: 17.10.2001
(73) Titulaire: Bruguiere, Jacques, 18320 Jouet-sur-l'Aubois (FR)
(72) Inventeur: Bruguiere, Jacques, 18320 Jouet-sur-l'Aubois (FR)
(74) Mandataire: Breese, Pierre
(86) Numéro de dépôt international: FR9901880
(87) Numéro de publication internationale: WO00006081

(56) Documents cités:
- US-A- 4 121 940
- US-A- 4 689 080
- CHEMICAL ABSTRACTS, vol. 99, no. 25, 19 décembre 1983 (1983-12-19) Columbus, Ohio, US; abstract no. 209714, TRYKOWSKI, JAN ET AL: "Antibacterial properties of new materials for filling root canals" XP002118427 & CZAS. STOMATOL. (1983), 36(4), 251-5 CODEN: CZSTA6;ISSN: 0011-4553,
- CHEMICAL ABSTRACTS, vol. 104, no. 24, 16 juin 1986 (1986-06-16) Columbus, Ohio, US; abstract no. 213197, LIMANOWSKA, HONORATA: "Evaluation of root canal filling materials using physical and biological methods" XP002118428 & POZNAN. ROCZ. MED. (1985), VOLUME DATE 1982-1983, 6-7, 27-43, 6 PLATES CODEN: POMDDV;ISSN: 0137-5350,
- DATABASE WPI Section Ch, Week 9729 Derwent Publications Ltd., London, GB; Class A96, AN 97-316433 XP002118429 & JP 09 124428 A (TAKAHASHI N), 13 mai 1997 (1997-05-13)

## Description

La présente invention concerne une nouvelle composition pâteuse, destinée à être utilisée comme matériau d'obturation lors d'une pulpotomie.

Le traitement usuel appliqué à une rage de dent comporte l'enlèvement de la totalité du nerf correspondant à la dent soignée. Cette opération, relativement fréquente est connue sous le nom de pulpectomie. Elle consiste à extirper le nerf de la dent soignée et à combler la cavité ainsi formée par un matériau d'obturation tel qu'une pâte antiseptique à base d'oxyde de zinc et d'eugénol ou une pâte à base de bakélite ou de la gutta condensée.

Ce type d'intervention ne présente pas de grande difficulté pour le praticien lorsqu'elle est pratiquée sur les dents aisément accessibles, telles que les incisives.

Par contre, lorsqu'elle est pratiquée sur une molaire, la pulpectomie se révèle être une opération longue et complexe, du fait de la variété de la forme des racines, de l'exiguïté de la cavité buccale et de la difficulté à anesthésier les racines de manière complète. Ainsi des études récentes (Information Dentaire n° 44 du 19 Décembre 1996-3535) ont montré que le taux d'échec des traitements endodontiques par pulpectomie s'élève à plus de 50 % dans le monde, Suisse, Suède et États-Unis compris. Ces échecs se traduisent par une infection qui se développe à l'extrémité de la racine. Ces échecs sont particulièrement gênants lorsque le matériau d'obturation utilisé est la bakélite. En cas d'échec du traitement il est impératif d'avoir à nouveau accès aux racines de la dent. Toutes les pâtes à canaux et en particulier la bakélite se révèlent particulièrement difficile à retirer.

En outre, des études portant sur les canaux (Information Dentaire No. 33, 28 Septembre 1995) ont montré que ceux-ci comportaient de nombreuses ramifications. Toutes ces ramifications ne peuvent être traitées lors d'une intervention et donc la pulpectomie ne s'effectue jamais sur la totalité du nerf.

De plus, la pratique de la pulpectomie s'accompagne de nombreux risques, parmi lesquels on peut citer l'inhalation par le patient d'instruments à canaux servant à extirper le nerf de sa cavité, ou encore le dépassement du matériau d'obturation sous les racines. Dans ce dernier cas, les complications peuvent être extrêmement graves, puisqu'elles vont de la sinusite fongique au maxillaire supérieur à la survenue de douleurs permanentes au maxillaire inférieur contre lesquelles aucune thérapeutique n'existe actuellement

Une alternative à la pulpectomie a, un temps, consisté en la pratique de la pulpotomie. Plutôt que de retirer la pulpe d'une dent dans sa totalité, la pulpotomie consiste à amputer sélectivement la partie coronaire de la pulpe d'une dent, contenue dans la chambre pulpaire, et à obturer la cavité ainsi formée par un matériau d'obturation classique.

La pulpotomie présente de nombreux avantages par rapport à la pulpectomie. Parmi ces avantages, on peut citer le gain de temps, un accès aisé à la partie du nerf à traiter, et une anesthésie plus légère mieux supportée par le patient. L'ensemble de ces facteurs entraîne la suppression des principaux risques qui accompagnent la pulpectomie et qui sont liés à l'utilisation des instruments à canaux et au dépassement de matériau d'obturation sous les racines.

Toutefois, les matériaux d'obturation utilisés jusqu'ici lors des pulpotomies n'ont pas donné satisfaction. En effet, ces matériaux d'obturation ne permettent pas d'éviter la dégénérescence et l'infection du nerf résiduel.

Par conséquent, du fait des nombreuses difficultés soulevées par l'inadaptation du matériau d'obturation utilisé, la pratique de la pulpotomie, malgré les avantages qu'elle peut présenter, a été abandonnée.

Le document brevet JP-A-09 124428 décrit une composition de soin dentaire comprenant du guaïacol, du paraformaldéhyde, de l'acide oxalique et de la formaline.

Un but de la présente invention vise donc à proposer une composition pâteuse utilisable comme matériau d'obturation lors d'une pulpotomie pratiquée sur une molaire, mais également sur une prémolaire ou une incisive, et palliant aux inconvénients de l'art antérieur.

Ce but est atteint selon la présente invention grâce à une nouvelle composition pâteuse, comprenant entre 10 et 15 % en poids d'un oxyde de métal, entre 3 et 6 % en poids d'au moins deux agents antiseptiques dont au moins l'un d'entre eux est un agent antiseptique relarguant à long terme, entre 0,5 et 2 % en poids de déxaméthasone, entre 25 et 35 % en poids de phénol, entre 20 et 30 % en poids de formaldéhyde, entre 20 et 30 % en poids de gaïacol et entre 0 et 1 % en poids d'acétate d'amyle.

De manière préférentielle, l'oxyde de métal utilisé dans la composition de l'invention est l'oxyde de zinc.

Par agent antiseptique relarguant à long terme, il faut entendre au sens de la présente invention, un agent antiseptique conservant ses propriétés de manière prolongée dans le temps. Ainsi, l'agent antiseptique relarguant à long terme utilisé dans la présente invention conserve ses propriétés durant plusieurs mois.

Dans une forme de réalisation préférée de l'invention l'agent antiseptique relarguant à long terme est le iodoforme.

De manière préférentielle, la composition de l'invention comprend 13 % en poids d'oxyde de zinc, 1 % en poids de trioxyméthylène, 3,5 % en poids d'iodoforme, 1% en poids de déxaméthasone, 30 % en poids de phénol, 27 % en poids de formaldéhyde, 24 % en poids de gaïacol et 0,5 % en poids d'acétate d'amyle.

La composition pâteuse objet de la présente invention peut être utilisée pour la préparation d'un matériau d'obturation utilisable lors d'une pulpotomie, dans le but d'obtenir un contact intime entre le matériau d'obturation et la chambre pulpaire, diminuant ainsi les risques d'infection des racines nerveuses résiduelles et prévenant leur dégénérescence.

La composition pâteuse de l'invention est préparée de manière extemporanée, par dispersion des différents composés solides sous forme de poudre dans les composés liquides, puis homogénéisation du mélange obtenu jusqu'à obtention de la consistance désirée. Pour être utilisée de façon optimale, la composition pâteuse de l'invention doit présenter une consistance crémeuse afin de présenter des propriétés de malléabilité propres à la rendre utilisable pour la préparation d'un matériau d'obturation utile lors d'une pulpotomie.

La composition pâteuse de l'invention peut également être utilisée dans le traitement d'une dent infectée. Il convient pour cela d'appliquer la composition de l'invention non seulement dans la chambre pulpaire mais aussi sur environ 1/3 de la hauteur des canaux partant de la chambre pulpaire. La composition ne doit en aucun cas passer au-delà de l'extrémité de la racine ou apex de la dent.

La présente invention concerne encore un pack comprenant notamment les composants constituant après mélange la composition pâteuse de l'invention ainsi qu'une notice décrivant les proportions respectives desdits composants adaptées à l'utilisation de ladite composition pour la préparation d'un matériau d'obturation utilisable lors d'une pulpotomie.

D'autres caractéristiques et avantages de la présente invention apparaîtront des exemples qui suivent.

### Exemple comparatif 1.

Une composition pâteuse peut être préparée avec les proportions suivantes :

A 25 mg d'une poudre formée de
- 80% en poids d'oxyde de zinc
- 5% en poids de trioxyméthylène :
- 15% en poids d'iodoforme
on ajoute 130 mg de la composition liquide suivante comprenant :
- 0,125% en poids de déxaméthazone
- 37% en poids de phénol
- 32 % en poids de formaldéhyde
- 30% en poids de gaïacol
- de l'acétate d'amyle : QSP 100 %

### Exemple 2.

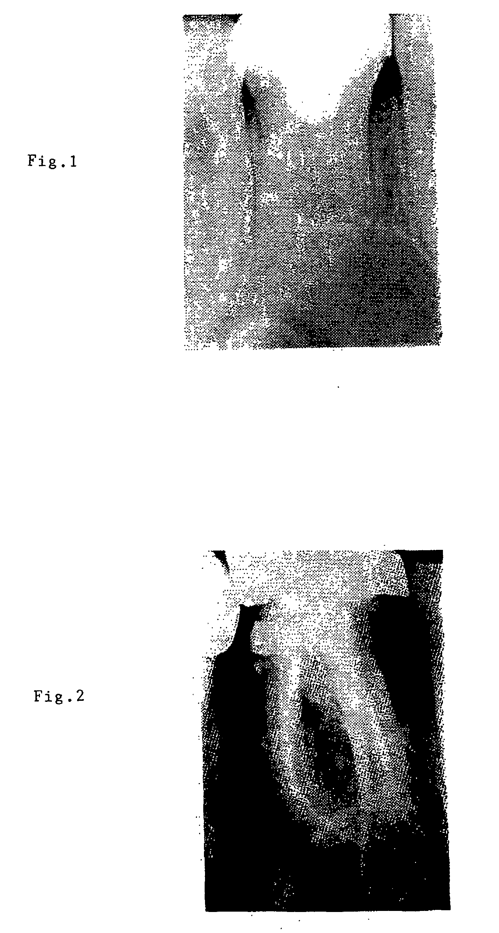

La figure 1 montre une radiographie de la racine d'une molaire sur laquelle une pulpotomie a été pratiquée en utilisant la composition pâteuse de l'invention. On constate que seule la chambre pulpaire a été obturée. Les canaux n'ont pas été touchés et restent vivants.

La figure 2 montre une radiographie de la racine d'une molaire sur laquelle une pulpectomie a été pratiquée en utilisant une composition pâteuse de l'art antérieur.

On remarque que le contenu de la chambre pulpaire ainsi que les canaux ont été extraits et leur cavité obturée par un matériau d'obturation. Du fait de la ramification collatérale des canaux qui rend leur extraction complète difficile, la pulpectomie est rarement totale.

### Exemple 3 :

Cet exemple illustre la préparation d'une composition selon la présente invention. 25 mg de la poudre formée des composants solides de l'invention sont mélangés à 130 mg d'une solution des composants liquides jusqu'à l'obtention d'une pâte de consistance crémeuse.

### Exemple 4 :

Cet exemple illustre la réalisation d'une pulpotomie à l'aide de la composition de l'invention.

La première étape d'une pulpotomie consiste à perforer le plafond de la chambre pulpaire à l'aide d'une fraise de type Zékrya chirurgicale. Le plafond est ensuite évincé à l'aide d'une fraise de type Zékrya endo. Finalement, la pulpe de la dent est retirée avec une grosse fraise diamentée poire ou boule. Il est important de réaliser ces étapes à grande vitesse afin que la pulpe soit sectionnée de manière nette et que les filets radiculaires ne soient pas arrachés.

Après que la pulpe ait été retirée, la composition obtenue à l'exemple 3 est injectée dans la chambre pulpaire à l'aide d'un bourre ciment. Un pansement provisoire est alors placé. On fait ensuite mordre progressivement mais fortement le patient sur un morceau de coton salivaire de façon à ce que la pâte épouse régulièrement et complètement toute la surface interne de la chambre pulpaire et notamment les orifices des canaux radiculaires. Lors d'une visite ultérieure, la dent sera définitivement obturée par un matériau classique en prenant soin de réaliser une obturation étanche.

### Exemple 5 :

L'exemple 5 illustre le traitement d'une dent infectée utilisant la composition préparée à l'exemple 3. Après avoir retiré la chambre pulpaire, les canaux sont élargis depuis leur départ de ladite chambre sur une hauteur de 1/3 au maximum avec des forets de Gates en progression de 1 à 6. Un réservoir est ainsi ménagé pour accueillir la composition. Une fois cette dernière introduite la dent est recouverte d'un pansement. On s'assure de la guérison de la dent en prenant une première radio 3 mois après l'intervention puis une seconde trois mois plus tard.

## Revendications

1. Composition pâteuse comprenant entre 10 et 15 % en poids d'un oxyde de métal, entre 3 et 6 % en poids d'au moins deux agents antiseptiques dont au moins l'un d'entre eux est un agent antiseptique relarguant à long terme, entre 0,5 et 2 % en poids de déxaméthasone, entre 25 et 35 % en poids de phénol, entre 20 et 30 % en poids de formaldéhyde, entre 20 et 30 % en poids de gaïacol et entre 0 et 1 % en poids d'acétate d'amyle.

2. Composition pâteuse selon la revendication 1, **caractérisée en ce que** l'oxyde de métal est l'oxyde de zinc.

3. Composition pâteuse selon l'une des revendications précédentes, **caractérisée en ce que** l'agent antiseptique relarguant à long terme est le iodoforme.

4. Composition pâteuse selon la revendication 1, **caractérisée en ce qu'**elle comprend 13 % en poids d'oxyde de zinc, 1 % en poids de trioxyméthylène, 3,5 % en poids d'iodoforme, 1% en poids de déxaméthasone, 30 % en poids de phénol, 27 % en poids de formaldéhyde, 24 % en poids de gaïacol et 0,5 % en poids d'acétate d'amyle.

5. Utilisation d'une composition pâteuse selon l'une des revendications précédentes pour la préparation d'un matériau d'obturation utilisable lors d'une pulpotomie.

6. Pack comprenant notamment les composants constituant après mélange la composition pâteuse selon l'une des revendications 1 à 4 ainsi qu'une notice décrivant les proportions respectives desdits composants adaptées à l'utilisation de ladite composition pour la préparation d'un matériau d'obturation utilisable lors d'une pulpotomie.

## Patentansprüche

1. Pastenartige Zusammensetzung, die zwischen 10 und 15 Gewichtsprozent eines Metalloxids enthält, zwischen 3 und 6 Gewichtsprozent mindestens zweier Antiseptika von denen mindestens eines ein langfristiges ausgesalzenes Antiseptikum ist, zwischen 0,5 und 2 Gewichtsprozent Dexamethason, zwischen 25 und 35 Gewichtsprozent Phenol, zwischen 20 und 30 Gewichtsprozent Formaldehyd, zwischen 20 und 30 Gewichtsprozent Guajakol und zwischen 0 und 1 Gewichtsprozent Amylacetat.

2. Pastenartige Zusammensetzung gemäß dem Patentanspruch 1, der **dadurch gekennzeichnet ist, dass** es sich bei dem Metalloxid um Zinkoxid handelt.

3. Pastenartige Zusammensetzung gemäß einem der vorherigen Patentansprüche, der **dadurch gekennzeichnet ist, dass** es sich bei dem langfristigen ausgesalzenen Antiseptikum um Iodoform handelt.

4. Pastenartige Zusammensetzung gemäß dem Patentanspruch 1, der **dadurch gekennzeichnet ist, dass** die Zusammensetzung 13 Gewichtsprozent Zinkoxid, 1 Gewichtsprozent Trioxidmethylen, 3,5 Gewichtsprozent Iodoform, 1 Gewichtsprozent Dexamethason, 30 Gewichtsprozent Phenol, 27 Gewichtsprozent Formaldehyd, 24 Gewichtsprozent Guajakol und 0,5 Gewichtsprozent Amylacetat enthält.

5. Einsatz einer pastenartigen Zusammensetzung gemäß einem der vorherigen Patentansprüche zur Vorbereitung eines Fugenmaterials, das bei einer Pulpotomie eingesetzt wird.

6. Großpackung, die vor allem die Bestandteile enthält, die nach der Mischung die pastenartige Zusammensetzung gemäß einem der Patentansprüche 1 bis 4 bilden sowie ein Erläuterungsschild, das die entsprechenden Mengen der besagten Bestandteile beschreibt, die an den Einsatz der besagten Zusammensetzung für die Vorbereitung eines Fugenmaterials angepasst sind, das bei einer Pulpotomie eingesetzt werden kann.

## Claims

1. Paste composition comprising between 10 and 15% by weight of a metal oxide, between 3 and 6% by weight of at least two antiseptic agents, at least one of which releases between 0.5 and 2% by weight of dexamethasone, between 25 and 35% by weight of phenol, between 20 and 30% by weight of formaldehyde, between 20 and 30% by weight of gaiacol and between 0 and 1% by weight of amyl acetate in the long term.

2. Paste composition according to claim 1, **characterised in that** the metal oxide is zinc oxide.

3. Paste composition according to one of the previous claims, **characterised in that** the antiseptic agent that releases the above mentioned products in the long term is iodoform.

4. Paste composition according to claim 1, **characterised in that** it comprises 13% by weight of zinc oxide, 1% by weight of trioxymethylene, 3.5% by weight of iodoform, 1% by weight of dexamethasone, 30% by weight of phenol, 27% by weight of formaldehyde, 24% by weight of gaiacol and 0.5% by weight of amyl acetate.

5. Use of a paste composition according to any of the previous claims for the preparation of a filling material that can be used in a pulpotomy.

6. Pack including particularly the components to be mixed together to form the paste composition according to one of claims 1 to 4, together with instructions describing the proportions of the said components adapted to use of the said composition for preparation of a filling material that can be used in a pulpotomy.
